# FASCICULE DE BREVET EUROPEEN

(11) **EP 3 755 445 B1**
(45) Date de publication et mention de la délivrance du brevet: **17.11.2021**
(21) Numéro de dépôt: 19707854.6
(22) Date de dépôt: 25.01.2019
(51) Int. Cl.: C07C 1/20, C07C 29/86, C07C 45/82, C07C 45/83, B01D 3/14, B01D 11/04, C07C 29/80

(54) **SÉPARATION SÉLECTIVE DES IMPURETÉS PRÉSENTES DANS UNE COUPE HYDROALCOOLIQUE PAR RECYCLAGE DANS UNE COLONNE D'EXTRACTION LIQUIDE-LIQUIDE**
SELEKTIVE TRENNUNG VON WÄSSERIG-ALKOHOLISCHER FRAKTION DURCH KREISLAUFFÜHRUNG IN EINER FLÜSSIG-FLÜSSIG-EXTRAKTION KOLONNE
SELECTIVE SEPARATION OF IMPURITIES FROM A HYDROALCOHOLIC FRACTION BY RECYCLING IN A COLUMN FOR LIQUID-LIQUID EXTRACTION

(30) Priorité: 22.02.2018 FR 1851549
(43) Date de publication de la demande: 30.12.2020
(73) Titulaire: IFP Energies nouvelles, 92852 Rueil-Malmaison Cedex (FR); COMPAGNIE GENERALE DES ETABLISSEMENTS MICHELIN, 63000 Clermont-Ferrand (FR)
(72) Inventeur: AUGIER, Frederic, 92852 RUEIL-MALMAISON CEDEX (FR); LEINEKUGEL LE COCQ, Damien, 92852 RUEIL-MALMAISON CEDEX (FR); DASTILLUNG, Rejane, 92852 RUEIL-MALAMAISON CEDEX (FR); BALZ, Pierre, 92852 RUEIL-MALMAISON CEDEX (FR)
(74) Mandataire: IFP Energies nouvelles
(86) Numéro de dépôt international: PCT/FR2019/050170
(87) Numéro de publication internationale: WO 2019/162586

(56) Documents cités:
- WO-A1-2018/001982
- FR-A1- 3 026 100
- FR-A1- 3 026 101

## Description

### CONTEXTE DE L'INVENTION ET ETAT DE LA TECHNIQUE

Les procédés de production de butadiène à partir d'éthanol ont été développés, en particulier, par des équipes russes sur la base des travaux de Lebedev dans les années 20 et par des équipes américaines durant la seconde guerre mondiale à partir des travaux d'Ostromilenski.

Dans ces procédés, la conversion par passe varie entre 30 et 60% environ, ce qui implique des recyclages importants de l'éthanol et de l'acétaldéhyde. De plus, une grande variété d'impuretés de différentes natures (hydrocarbures saturés, insaturés, aromatiques, produits oxygénés tels que alcools, cétones, aldéhydes, phénols, acides, esters, éthers) et ayant des masses molaires très différentes est produite (entre 50 et 10 000 g/mol).

En conséquence, il est donc nécessaire de mettre en place un enchaînement d'opérations unitaires dans le but d'éliminer le maximum d'impuretés en perdant le moins possible d'éthanol et d'acétaldéhyde afin d'augmenter le rendement aux bornes du procédé, d'éviter l'accumulation de ces impuretés dans le procédé et éviter leur réactivité avec des équivalents de charge, voire un effet d'empoisonnement des catalyseurs. D'un point de vue économique, il est primordial de diminuer le coût de production du butadiène, ce qui nécessite de :
- perdre le moins possible d'éthanol et d'acétaldéhyde,
- ne pas recycler d'impuretés dans les réacteurs qui induiraient une chute de sélectivité en butadiène ou qui s'accumuleraient à des niveaux non acceptables, nécessitant une purge et donc des pertes en éthanol et acétaldéhyde.

À la sortie des réacteurs catalytiques, l'effluent produit composé de butadiène, d'acétaldéhyde, d'eau, d'éthanol et d'impuretés subit plusieurs opérations unitaires afin de séparer les sous-produits les plus volatils des sous-produits moins volatils..

Parmi les sous-produits les plus volatils, on peut citer l'hydrogène, le monoxyde de carbone, le dioxyde de carbone, les alcanes et les oléfines en C₁-C₄. Il est primordial d'éliminer ces sous-produits de l'effluent riche en butadiène afin d'obtenir un produit aux spécifications.

Parmi les sous-produits moins volatils, on peut citer l'acétone, le diéthyléther, le butanol, le butanal, le butènol, le butanone, l'éthyle acétate, le crotonaldéhyde, l'acide acétique, le méthanol et d'autres impuretés apolaires. D'autres sous-produits sont générés, en plus faible quantité dans la zone réactionnelle. Dans la suite du document, on désignera par « impuretés » cet ensemble de composés hydrocarbonés et/ou oxygénés.

Dans les premiers schémas de procédé des équipes américaines, l'éthanol, l'acétaldéhyde, l'eau et les sous-produits liquides étaient séparés par un enchaînement de trois colonnes à distiller (brevet US 2,403,742). L'effluent riche en éthanol, acétaldéhyde, eau et sous-produits liquides alimente une première colonne à distiller dans laquelle un effluent riche en acétaldéhyde est séparé du reste de l'effluent. Une seconde colonne à distiller permet de séparer les sous-produits liquides d'un effluent riche en éthanol et eau. La dernière colonne à distiller permet de séparer l'éthanol de l'eau. La majeure partie des brevets de procédé déposés dans la période 1940-1960 par les sociétés Carbide & Carbon ou Koppers (US 2,403,743 ; US 2,393,381 ; US 2,395,057 et US 2,439,587) visent à améliorer cette partie du schéma. Cependant, la séparation n'est pas totale et des impuretés se retrouvent dans l'effluent riche en éthanol avec lequel elles sont recyclées vers les réacteurs catalytiques.

Dans les demandes FR 3 026 100 (A1) et FR 3 026 101 (A1), l'élimination des impuretés liquides se fait par extraction liquide-liquide. L'effluent composé d'éthanol, d'acétaldéhyde, d'eau et d'impuretés alimente une section d'extraction liquide-liquide composée d'une colonne de lavage (L) de l'effluent par un solvant organique et d'une colonne de contre-lavage (CL) à l'eau. La colonne de lavage (L) est alimentée en fond par le solvant de lavage qui a pour but de laver à contre-courant la charge. Le solvant de lavage utilisé pour cette opération unitaire peut contenir des hydrocarbures saturés et/ou insaturés et /ou aromatiques, avantageusement constitué d'un mélange d'hydrocarbures ayant entre 6 et 40 atomes de carbone. Il peut être une coupe gazole ou kérosène désulfurée ou bien encore une coupe d'hydrocarbures produite par une unité de type Fischer-Tropsh. Le solvant organique utilisé est ensuite régénéré par distillation et réutilisé. A la sortie de la colonne de lavage (L), l'extrait est composé majoritairement du solvant de lavage, de sous-produits extraits et d'une faible quantité d'éthanol et d'acétaldéhyde. Cet extrait est ensuite lavé à l'eau dans la colonne de contre-lavage (CL) dans le but de ré-extraire l'éthanol et l'acétaldéhyde et ainsi minimiser les pertes en éthanol et acétaldéhyde.

Dans les demandes FR 3 026 100 (A1) et FR 3 026 101 (A1), l'extraction liquide-liquide permet d'éliminer une forte proportion des impuretés, notamment des impuretés apolaires et peu polaires, et des huiles brunes. Cependant, certaines impuretés hydrocarbonées, en particulier les impuretés hydrocarbonées partiellement polaires, comme par exemple le butanol, ne sont que très peu extraites lors de cette extraction liquide-liquide. Le raffinat sortant en fond de section d'extraction, outre l'éthanol, l'acétaldéhyde et l'eau, contient donc encore de nombreuses impuretés, comme par exemple le butanol ou encore le méthanol, l'acétate d'éthyle.

De plus, dans les demandes FR 3 026 100 (A1) et FR 3 026 101 (A1), le raffinat sortant en fond de section d'extraction est ensuite envoyé vers une section de distillation, à 2 ou 3 colonnes, dont le but est de séparer les composés éthanol, acétaldéhyde, et eau, cette dernière concentrant une partie importante des impuretés encore présentes. Les impuretés, ayant une température d'ébullition comprise entre celles des réactifs, l'éthanol et l'acétaldéhyde, et celle de l'eau, c'est-à-dire entre environ 20°C et environ 100°C peuvent potentiellement s'accumuler dans l'une ou l'autre des colonnes à distiller. Cette accumulation peut provoquer plusieurs phénomènes non désirés voire néfastes au bon fonctionnement des colonnes de distillation. Elle peut notamment provoquer une démixtion locale (création d'une deuxième phase liquide) qui diminue l'efficacité de séparation ou encore une modification des équilibres thermodynamiques entres les espèces présentes dans la zone d'accumulation. Par exemple, le butanol présent dans le raffinat en sortie d'extraction liquide-liquide, et dans la mesure où le butanol forme un azéotrope avec l'eau avec une température d'ébullition d'environ 92°C, a tendance à s'accumuler dans la colonne de distillation éthanol/eau.

Une solution simple à ce problème consisterait à effectuer un soutirage, continu ou discontinu, au niveau de la colonne à distiller afin de purger l'impureté considérée. Or ce soutirage contiendrait l'impureté diluée dans les composés d'intérêt, notamment l'éthanol et l'acétaldéhyde. Il pourrait donc représenter une perte en espèces « nobles », d'intérêt industriel.

Un objectif de l'invention est d'améliorer la séparation des impuretés hydrocarbonées, en particulier des impuretés hydrocarbonées partiellement polaires, qui sont des sous-produits de la réaction catalytique de transformation de l'éthanol en butadiène tout en optimisant la récupération de l'éthanol et de l'acétaldéhyde. L'intérêt de l'invention réside dans le fait que cette optimisation est envisagée sans multiplier les étapes de traitement des effluents. L'invention s'applique, en particulier, avantageusement à l'effluent éthanol / acétaldéhyde / eau dans les procédés des demandes FR 3 026 100 (A1) et FR 3 026 101 (A1).

### RESUME DE L'INVENTION

La présente invention concerne un procédé de traitement par extraction liquide-liquide et distillation d'une charge liquide comprenant au moins de l'éthanol, de l'eau et de l'acétaldéhyde et au moins une impureté hydrocarbonée ayant une température d'ébullition comprise entre 20 et 100°C à pression atmosphérique ou générant, avec au moins l'un des composés de la charge liquide et/ou avec le solvant organique d'extraction et/ou avec le solvant aqueux de contre-lavage, un azéotrope dont la température d'ébullition est comprise entre 20 et 100°C à pression atmosphérique, et un coefficient de partage compris entre 0,1 et 5 en tout point de la colonne de contre-lavage (CL) de la section d'extraction, comprenant :
a) une étape d'extraction liquide-liquide comprenant une section d'extraction comprenant une colonne de lavage (L) et une colonne de contre-lavage (CL), ladite section d'extraction étant alimentée en ladite charge liquide en un point intermédiaire situé entre la tête de la colonne de lavage (L) et le fond de la colonne de contre-lavage (CL),
   ladite colonne de lavage (L) étant alimentée en tête par le flux issu du fond de la colonne de contre-lavage (CL) et en fond par un flux d'un solvant organique d'extraction,
   ladite colonne de contre-lavage (CL) étant alimentée en tête par un flux d'un solvant aqueux de contre-lavage et en fond par le flux issu de la tête de la colonne de lavage (L),
   ladite section d'extraction comprenant au moins une injection (F2) de la coupe soutirée à l'étape b), ladite injection (F2) étant située dans la moitié supérieure de la colonne de contre-lavage (CL),
   ladite section d'extraction produisant en fond de colonne de lavage (L) un raffinat comprenant au moins de l'eau, de l'éthanol, de l'acétaldéhyde et une partie de ladite impureté hydrocarbonée et en tête de colonne de contre-lavage (CL) un effluent d'extraction comprenant au moins une partie de ladite impureté hydrocarbonée ;
b) une étape de distillation comprenant :
   ∘ au moins une étape de séparation de l'acétaldéhyde comprenant une section de séparation de l'acétaldéhyde, composée au moins d'une colonne de distillation (D1) alimentée par le raffinat issu de l'étape a) dans une zone intermédiaire de la colonne (D1) et produisant en tête un effluent riche en acétaldéhyde et en fond un effluent eau/éthanol ; et
   ∘ au moins une étape de séparation de l'éthanol comprenant une section de séparation de l'éthanol, composée au moins d'une colonne de distillation (D2) alimentée par l'effluent eau/éthanol issu du fond de la colonne (D1) dans une zone intermédiaire de la colonne (D2), et produisant en tête un effluent riche en éthanol et en fond un effluent riche en eau,
   ∘ au moins un soutirage latéral sur la colonne (D1) ou la colonne (D2) d'une coupe comprenant au moins l'un des composés éthanol ou acétaldéhyde, à une fraction massique d'au moins 10% de la masse totale de la coupe soutirée, et ladite impureté hydrocarbonée, à une fraction massique comprise entre 0,5% et 50% de la masse totale de la coupe soutirée, le soutirage latéral étant situé dans une zone intermédiaire de la colonne (D1) ou (D2).

Le procédé selon l'invention consiste à extraire partiellement de la charge une impureté hydrocarbonée difficile à séparer, par extraction-distillation classique, en une seule passe, ladite impureté hydrocarbonée, de préférence à une teneur faible dans la charge à traiter, ayant une température d'ébullition intermédiaire, ou formant, avec au moins l'un des composés de la charge liquide, notamment avec l'eau, l'éthanol et/ou l'acétaldéhyde, en particulier avec l'eau, et/ou avec le solvant organique d'extraction et/ou le solvant aqueux de contre-lavage de l'étape a) du procédé selon l'invention, un azéotrope ayant une température d'ébullition intermédiaire, par rapport aux températures d'ébullition des composés d'intérêt de la charge (éthanol, acétaldéhyde et eau), et ayant une affinité faible avec le solvant d'extraction.

Il a été ainsi découvert, de manière surprenante, qu'il est possible d'extraire au moins partiellement cette impureté hydrocarbonée, en particulier partiellement polaire, difficile à séparer, par un système de soutirage et de recyclage d'une coupe contenant ladite impureté hydrocarbonée, vers la colonne de contre-lavage. Les inventeurs ont en effet mis en évidence que, lorsque l'impureté hydrocarbonée possède une affinité avec le solvant d'extraction faible mais supérieure à celle de l'éthanol et celle de l'acétaldéhyde, le fait de réinjecter la coupe soutirée contenant l'impureté hydrocarbonée difficile à séparer dans la partie haute de la colonne de contre-lavage, permet d'extraire au moins une fraction de l'impureté hydrocarbonée réinjectée, sans perte significative d'éthanol et d'acétaldéhyde, et pour un coût modéré.

Avantageusement, le procédé selon l'invention permet de produire un effluent concentré en acétaldéhyde et un effluent concentré en éthanol, sans perte significative de ces composés, et tout en limitant la consommation d'énergie.

L'invention s'applique avantageusement au traitement des effluents hydroalcooliques issus de la conversion de l'éthanol en butadiène. En particulier, le procédé selon l'invention s'applique avantageusement à l'étape d'élimination des impuretés hydrocarbonées et des huiles brunes, décrite par exemple dans les demandes FR 3 026 100 (A1) et FR 3 026 101 (A1), étape d'élimination qui comprend entre autre une section d'extraction liquide-liquide et une section de distillation.

Les effluents produits selon l'invention, en particulier l'effluent concentré en acétaldéhyde et l'effluent concentré en éthanol, pourront être recyclés comme réactifs dans le procédé de conversion de l'éthanol en butadiène, dit procédé Lebedev, pour produire du butadiène et ainsi améliorer le rendement global de production de butadiène.

### DESCRIPTION DE L'INVENTION

Selon l'invention, on entend par « coefficient de partage » (M) d'une espèce i en un point de l'extracteur, le ratio entre la fraction massique de l'espèce i considérée dans la phase organique d'extraction (Yi) et la fraction massique de cette même espèce i dans la phase aqueuse (Xi). La phase organique d'extraction comprend le solvant organique d'extraction dans lequel sont extraites notamment les impuretés apolaires ou peu polaires et au moins en partie l'impureté hydrocarbonée partiellement polaire considérée. Le coefficient de partage peut varier sensiblement le long de l'extracteur. Selon l'invention, on entend par extracteur, l'ensemble des colonnes de lavage (L) et contre-lavage (CL), dans lesquelles circulent à contre-courant les flux de phase organique et de phase aqueuse. Le coefficient de partage considéré selon l'invention pour apprécier l'impureté hydrocarbonée extraite par le procédé selon l'invention est le coefficient de partage de cette impureté hydrocarbonée en tout point de la colonne de contre-lavage (CL) de la section d'extraction de l'étape a) du procédé selon l'invention.

Selon l'invention, on entend par « impureté hydrocarbonée partiellement polaire » ou « impureté hydrocarbonée », un composé hydrocarboné, autre que l'éthanol et l'acétaldéhyde, de préférence présent en faible quantité dans la charge liquide (notamment à une teneur inférieure ou égale à 5% poids de la charge à traiter), et ayant une affinité avec la phase organique d'extraction comprenant le solvant organique d'extraction faible mais supérieure à celles de l'éthanol et de l'acétaldéhyde, en tout point de la colonne de contre-lavage (CL) de la section d'extraction du procédé selon l'invention. Le coefficient de partage de l'impureté hydrocarbonée considérée en tout point de la colonne de contre-lavage (CL) de la section d'extraction est aussi, avantageusement, inférieur à une valeur au-delà de laquelle l'impureté hydrocarbonée peut être considérée comme étant apolaire ou peu polaire et donc extraite par le solvant organique d'extraction lors de son premier passage dans la colonne de lavage (L). Cette impureté apolaire ou peu polaire ne devrait donc pas être présente dans le raffinat en pied de colonne de lavage (L). Ainsi, l'invention est pertinente pour extraire, au moins partiellement, les impuretés hydrocarbonées ayant un coefficient de partage compris entre 0,1 et 5 en tout point de la colonne de contre-lavage (CL) de la section d'extraction de l'étape a) du procédé selon l'invention.

Selon la présente invention, l'expression « compris entre ... et ... » signifie que les valeurs limites de l'intervalle sont incluses dans la gamme de valeurs décrite. Si tel n'était pas le cas et que les valeurs limites n'étaient pas incluses dans la gamme décrite, une telle précision sera apportée par la présente invention.

Ainsi, la présente invention consiste en un procédé de traitement par extraction liquide-liquide et distillation d'une charge liquide comprenant au moins de l'éthanol, de l'eau et de l'acétaldéhyde et au moins une impureté hydrocarbonée ayant une température d'ébullition comprise entre 20°C et 100°C à pression atmosphérique, ou générant, avec au moins l'un des composés de la charge liquide et/ou avec le solvant organique d'extraction et/ou avec le solvant aqueux de contre-lavage, un azéotrope dont la température d'ébullition est comprise entre 20 et 100°C à pression atmosphérique, et un coefficient de partage compris entre 0,1 et 5 en tout point de la colonne de contre-lavage (CL) de la section d'extraction, comprenant :
a) une étape d'extraction liquide- liquide comprenant une section d'extraction comprenant une colonne de lavage (L) et une colonne de contre-lavage (CL), ladite section d'extraction étant alimentée en ladite charge liquide en un point intermédiaire situé entre la tête de la colonne de lavage (L) et le fond de la colonne de contre-lavage (CL),
   ladite colonne de lavage (L) étant alimentée en tête par le flux issu du fond de la colonne de contre-lavage (CL) et en fond par un flux d'un solvant organique d'extraction, de préférence à un débit massique compris entre 25% et 200% du débit massique du flux d'alimentation en ladite charge liquide,
   ladite colonne de contre-lavage (CL) étant alimentée en tête par un flux d'un solvant aqueux, de préférence à un débit massique compris entre 10% et 80% du débit massique du flux d'alimentation en ladite charge liquide, et en fond par le flux issu de la tête de la colonne de lavage (L),
   ladite section d'extraction comprenant au moins une injection (F2) de la coupe soutirée à l'étape b), de préférence à un débit massique compris entre 0,1% et 10% du débit massique du flux d'alimentation en ladite charge liquide, ladite injection (F2) étant située dans la moitié supérieure de la colonne de contre-lavage (CL),
   ladite section d'extraction produisant en fond de colonne de lavage (L) un raffinat comprenant au moins de l'eau, de l'éthanol, de l'acétaldéhyde et une partie de ladite impureté hydrocarbonée et en tête de colonne de contre-lavage (CL) un effluent d'extraction comprenant au moins une partie de ladite impureté hydrocarbonée ;
b) une étape de distillation comprenant :
   ∘ au moins une étape de séparation de l'acétaldéhyde comprenant une section de séparation de l'acétaldéhyde, composée au moins d'une colonne de distillation (D1) alimentée par le raffinat issu de l'étape a) dans une zone intermédiaire de la colonne (D1) et produisant en tête un effluent riche en acétaldéhyde et en fond un effluent eau/éthanol ; et
   ∘ au moins une étape de séparation de l'éthanol comprenant une section de séparation de l'éthanol, composée au moins d'une colonne de distillation (D2) alimentée par l'effluent eau/éthanol issu du fond de la colonne (D1) dans une zone intermédiaire de la colonne (D2), et produisant en tête un effluent riche en éthanol et en fond un effluent riche en eau,
   ∘ au moins un soutirage latéral sur la colonne (D1) ou la colonne (D2) d'une coupe comprenant au moins l'un des composés éthanol ou acétaldéhyde, à une fraction massique d'au moins 10% de la masse totale de la coupe soutirée, et ladite impureté hydrocarbonée, à une fraction massique comprise entre 0,5% et 50% de la masse totale de la coupe soutirée, le soutirage latéral étant situé dans une zone intermédiaire de la colonne (D1) ou (D2).

De préférence, le procédé de traitement selon l'invention consiste en l'étape a) d'extraction liquide-liquide et en l'étape b) de distillation, précédemment citées. De manière encore plus préférée, le procédé de traitement selon l'invention consiste en l'étape a) d'extraction liquide-liquide et en l'étape b) de distillation, cette dernière consistant en l'étape de séparation de l'acétaldéhyde comprenant la section de séparation de l'acétaldéhyde composée au moins de la colonne (D1), en l'étape de séparation de l'éthanol comprenant la section de séparation de l'éthanol composée au moins de la colonne (D2), et en le soutirage latéral.

### La charge

Le procédé selon l'invention s'applique au traitement de charges liquides comprenant au moins de l'éthanol, de l'eau et de l'acétaldéhyde et comprenant au moins une impureté hydrocarbonée partiellement polaire. Il s'applique avantageusement au traitement des effluents hydroalcooliques du procédé de conversion de l'éthanol en butadiène, c'est-à-dire du procédé Lebedev.

La charge liquide traitée par le procédé selon l'invention est de préférence une solution, suspension ou émulsion.

La charge liquide à traiter peut comprendre une ou plusieurs impuretés hydrocarbonées partiellement polaires, de préférence en faible quantité en particulier par rapport aux composés « nobles » (ou composés d'intérêt industriel), c'est-à-dire l'éthanol et l'acétaldéhyde, et à l'eau. L'impureté hydrocarbonée considérée, c'est-à-dire l'impureté hydrocarbonée partiellement polaire à extraire, ou chacune des impuretés hydrocarbonées considérées, représente notamment moins de 5% poids, en particulier moins de 2% poids, plus particulièrement moins de 1% poids de la charge liquide à traiter.

Avantageusement, l'impureté hydrocarbonée considérée a une température d'ébullition comprise entre celles des composés majoritaires de la charge traitée (éthanol, eau, acétaldéhyde), c'est-à-dire comprise entre 20°C et 100°C à pression atmosphérique. L'impureté hydrocarbonée considérée peut générer, avec au moins l'un des composés de la charge liquide, notamment avec l'eau, l'éthanol et/ou l'acétaldéhyde, en particulier avec l'eau, et/ou avec le solvant organique d'extraction de l'étape a) du procédé selon l'invention et/ou le solvant aqueux de contre-lavage de l'étape a) du procédé selon l'invention, un azéotrope dont la température d'ébullition à pression atmosphérique est dans cette même gamme de température, c'est-à-dire comprise entre 20°C et 100°C. Dans ce cas de formation d'un azéotrope, avec au moins l'un des composés de la charge liquide et/ou avec le solvant organique d'extraction et/ou le solvant aqueux de contre-lavage, c'est la température d'ébullition de l'azéotrope qui est considérée comme limitante et qui est donc prise en compte.

Selon l'invention, l'impureté hydrocarbonée considérée a également des valeurs de coefficient de partage compris entre 0,1 et 5 en tout point de la colonne de contre-lavage (CL) de la section d'extraction, de préférence compris entre 0,1 et 3 et préférentiellement entre 0,2 et 2. Elle a ainsi une affinité faible avec la phase organique d'extraction comprenant le solvant organique d'extraction mais supérieure à celles de l'éthanol et de l'acétaldéhyde (inférieures ou égales à 0.05) avec cette même phase organique d'extraction. Les impuretés hydrocarbonées, dont le coefficient de partage en tout point de de la colonne de contre-lavage (CL) de la section d'extraction est supérieur à 5, sont extraites par simple lavage au solvant organique d'extraction ; le procédé selon l'invention qui comporte un système de soutirage et recyclage d'une coupe de distillation vers la section de lavage/contre-lavage n'est pas nécessaire pour extraire ces impuretés à haut coefficient de partage (> 5).

La(les) impureté(s) hydrocarbonée(s), que l'on cherche à extraire par le procédé selon l'invention, est(sont), par exemple, le butanol, l'éthylacétate, ou toute autre impureté hydrocarbonée co-produite lors de la conversion de l'éthanol en butadiène et présentant une température d'ébullition comprise entre 20°C et 100°C à pression atmosphérique, ou générant , avec au moins l'un des composés de la charge liquide et/ou avec le solvant organique d'extraction et/ou avec le solvant aqueux de contre-lavage, un azéotrope dont la température d'ébullition est comprise entre 20 et 100°C à pression atmosphérique, et ayant un coefficient de partage compris entre 0,1 et 5 en tout point de la colonne de contre-lavage (CL) de la section d'extraction.

### L'étape a) d'extraction

Conformément à l'invention, le procédé de traitement comprend une étape d'extraction liquide-liquide comprenant une section d'extraction avec au moins une colonne de lavage (L) et une colonne de contre-lavage (CL). La section d'extraction produit en fond de colonne de lavage (L) un raffinat comprenant au moins de l'eau, de l'éthanol, de l'acétaldéhyde et une partie de l'impureté hydrocarbonée considérée et en tête de colonne de contre-lavage (CL) un effluent d'extraction comprenant au moins une partie de l'impureté hydrocarbonée considérée.

La colonne de lavage (L) est alimentée en tête par le flux issu du fond de la colonne de contre-lavage (CL) et en fond par un flux de solvant organique d'extraction ; la colonne de contre-lavage (CL) est quant à elle alimentée en tête par un flux de solvant aqueux de contre-lavage et en fond par le flux issu de la tête de la colonne de lavage (L).

Selon l'invention, la section d'extraction est alimentée en la charge liquide (F1) à traiter en un point intermédiaire situé entre la tête de la colonne de lavage (L) et le fond de la colonne de contre-lavage (CL). De préférence, la charge liquide (F1) à traiter alimente la section d'extraction en amont de la tête de colonne de lavage (L), pour subir, d'abord, le lavage à contre-courant par le solvant organique d'extraction.

Ainsi, de façon tout à fait classique, la charge à traiter selon l'invention est lavée à contre-courant par le solvant organique d'extraction dans la colonne de lavage (L). Les impuretés apolaires ou peu polaires (impuretés ayant un coefficient de partage supérieur à 5 en tout point de la colonne de contre-lavage (CL) de la section d'extraction) sont extraites dans le solvant de lavage. Le flux organique est ensuite lavé à contre-courant par le solvant aqueux permettant ainsi de contre-extraire du solvant organique les composés « nobles » (c'est-à-dire les composés d'intérêt), éthanol et acétaldéhyde, éventuellement dissouts dans le solvant organique. Ce principe de lavage/contre-lavage permet de minimiser les pertes en éthanol et acétaldéhyde lors de cette étape.

Conformément à l'invention, la section d'extraction comprend au moins une injection (F2) de la(des) coupe(s) soutirée(s) à l'étape b) du procédé selon l'invention. Avantageusement, l'injection (ou les injections) (F2) est(sont) située(s) dans la moitié supérieure de la colonne de contre-lavage (CL), de manière préférée dans la partie haute de la colonne de contre-lavage (CL) tel que la distance entre la tête de la colonne de contre-lavage (CL) et l'injection (F2) est égale à une longueur comprise entre 10 et 30% de la longueur totale de la colonne de contre-lavage (CL), la position 0% signifiant que l'injection F2 se situe en tête de la colonne de contre-lavage (CL) et la position 100% que l'injection F2 se situe en fond de colonne de contre-lavage (CL).

Avantageusement, le flux soutiré et recyclé (F2), ou chaque flux soutiré et recyclé (F2), est injecté à un débit massique compris entre 0,1% et 10% du débit massique de la charge liquide alimentant la section d'extraction selon l'invention. Le flux soutiré et recyclé (F2) comprend l'impureté hydrocarbonée considérée à une concentration massique avantageusement comprise entre 0,5% et 50%, de préférence entre 1% et 30%, par rapport à la masse du flux recyclé (F2).

Le recyclage de la coupe soutirée à l'étape b) du procédé selon l'invention, notamment vers un point d'injection (F2) précis sur la colonne de contre-lavage (CL), permet l'extraction au moins partielle de l'impureté hydrocarbonée considérée, par exemple le butanol, avec une efficacité d'extraction satisfaisante, notamment supérieure à 10% poids, en particulier comprise entre 15% et 35% poids, par exemple de l'ordre de 20 à 30% poids, pour des pertes en éthanol inférieures à 0,1% poids, avantageusement inférieures ou égales à 0,05% poids, et des pertes en acétaldéhyde inférieures à 0,1% poids, avantageusement inférieures ou égales à 0,05% poids, ces pertes jugées étant considérées comme faibles voire négligeables.

Selon l'invention, l'efficacité d'extraction correspond au rapport entre la quantité massique d'impureté hydrocarbonée considérée extraite dans l'effluent d'extraction sortant en tête de colonne de contre-lavage (CL), et la quantité massique de ladite impureté hydrocarbonée entrant dans la section d'extraction (c'est-à-dire présente dans charge à traiter). La perte en éthanol correspond au rapport entre la quantité massique d'éthanol extrait dans l'effluent d'extraction sortant en tête de colonne de contre-lavage (CL) et la quantité massique d'éthanol entrant dans la section d'extraction (c'est-à-dire présente dans la charge à traiter).

L'extraction partielle de l'impureté hydrocarbonée considérée, par exemple le butanol, est suffisante pour stabiliser sa teneur dans la boucle de recyclage et pour purger la totalité du de l'impureté hydrocarbonée considérée produite dans les sections réactionnelles du procédé de conversion de l'éthanol en butadiène, et ceci pour un coût raisonnable.

Selon l'invention, le débit massique en solvant organique d'extraction (le solvant de lavage), est avantageusement compris entre 25% et 200%, de préférence entre 30% et 150%, du débit massique de la charge liquide alimentant la section d'extraction selon l'invention. Le débit massique de solvant aqueux de contre-lavage est avantageusement compris entre 10% et 80%, de préférence entre 10% et 50%, du débit massique de la charge liquide alimentant la section d'extraction selon l'invention.

Les critères de choix du solvant organique d'extraction sont bien connus de l'Homme du métier, comme par exemple l'affinité avec les impuretés à extraire, les propriétés physiques du solvant organique telles que la densité qui doit être suffisamment différente de celle du solvant de contre-lavage, la viscosité qui ne doit pas être trop élevée (de préférence inférieure ou égale à 5 cPs à température ambiante), une température d'ébullition suffisamment éloignées de celles des impuretés à extraire, le coût du solvant organique, etc. Dans le cas particulier de la charge liquide à traiter, issue du procédé de production du butadiène à partir de l'éthanol (procédé Lebedev), l'homme du métier sait comment choisir le solvant organique d'extraction le plus adéquate en fonction notamment des impuretés apolaires ou peu polaires contenues dans la charge à traiter, comme par exemple le diéthyléther. De préférence, le solvant organique d'extraction est choisi parmi les hydrocarbures saturés et/ou insaturés et/ou aromatiques. Le solvant organique d'extraction est avantageusement un hydrocarbure ou un mélange d'hydrocarbures ayant entre 6 et 40 atomes de carbone, de préférence entre 10 et 20 atomes de carbone, comme par exemple l'hexadécane. Il peut également s'agir d'une coupe gazole ou kérosène désulfurée ou bien encore d'une coupe d'hydrocarbures produite par une unité de type Fischer-Tropsh.

Selon l'invention, le solvant aqueux de contre-lavage est un solvant aqueux neutre, acide ou basique. De préférence, le solvant aqueux utilisé pour le contre-lavage dans le procédé est l'eau ou l'eau acidifiée dont le pH est compris entre 0,5 et 5.

Dans un mode particulier de l'invention, la colonne de lavage comprend entre 2 et 10 étages théoriques, de préférence entre 3 et 7 étages théoriques et notamment 5 étages théoriques, et la colonne de contre-lavage comprend entre 1 et 6 étages théoriques, de préférence entre 2 et 4 étages théoriques.

Avantageusement, l'étape d'extraction liquide-liquide est opérée, dans chacune des colonnes de lavage et contre-lavage, à une température comprise entre 10°C et 70°C, de préférence entre 20°C et 55°C, et à une pression comprise entre 0,1 MPa et 0,5 MPa. Les temps de séjour sont typiquement compris entre 0,5 et 10h, de préférence entre 0,5 et 6h, dans la colonne de lavage et compris entre 0,5 et 6h, de préférence entre 1 et 3h, dans la colonne de contre-lavage.

Avantageusement, le solvant organique d'extraction peut être ensuite récupéré en sortie de section d'extraction puis régénéré par exemple par distillation pour être réutilisé dans la colonne de lavage.

### L'étape b) de distillation

Conformément à l'invention, le procédé de traitement comprend une étape de distillation comprenant au moins une étape de séparation de l'acétaldéhyde comprenant une section de séparation de l'acétaldéhyde et au moins une étape de séparation de l'éthanol comprenant une section de séparation de l'éthanol.

La section de séparation de l'acétaldéhyde comprend au moins une colonne de distillation (D1). La colonne (D1) est alimentée dans une zone intermédiaire en raffinat issu de l'étape a), plus précisément issu du fond de la colonne de lavage (L) de l'étape a), ledit raffinat comprenant au moins de l'eau, de l'éthanol, de l'acétaldéhyde et de l'impureté hydrocarbonée considérée. La section de séparation de l'acétaldéhyde produit en tête de colonne (D1) un effluent riche en acétaldéhyde et en fond de colonne (D1) un effluent eau/éthanol.

L'effluent riche en acétaldéhyde produit en tête de colonne (D1) comprend au moins 50% poids, de préférence au moins 70% poids et préférentiellement 80% poids d'acétaldéhyde. Il peut ainsi être recyclé vers la section réactionnelle de production du butadiène où l'acétaldéhyde pourra être utilisé comme réactif.

L'effluent eau/éthanol produit en fond de colonne (D1) peut comprendre l'impureté hydrocarbonée considérée, par exemple le butanol.

La section de séparation de l'éthanol comprend au moins une colonne de distillation (D2). La colonne (D2) est alimentée en effluent eau/éthanol issu du fond de la colonne (D1) dans une zone intermédiaire. La section de séparation de l'éthanol produit en tête de colonne (D2) un effluent riche en éthanol et en fond de colonne (D2) un effluent riche en eau.

L'effluent riche en éthanol produit en tête de colonne (D2) comprend au moins 70% poids, de préférence au moins 80% poids d'éthanol. Il comprend en outre de l'eau. L'effluent riche en éthanol peut ainsi être recyclé vers la section réactionnelle dans laquelle l'éthanol sera consommé comme réactif.

L'effluent riche en eau comprend essentiellement de l'eau. En particulier, l'effluent riche en eau comprend de l'eau et moins de 10 ppm massique, de préférence moins de 5 ppm massique, d'éthanol, moins de 5 ppm massique d'acétaldéhyde, et moins de 5 ppm massique, de préférence moins de 1 ppm, d'impureté hydrocarbonée partiellement polaire. Selon l'invention, l'étape de distillation comprend au moins un soutirage latéral sur la colonne (D1) ou (D2). La coupe soutirée est recyclée ensuite vers la colonne de contre-lavage (CL) de l'étape a).

Le soutirage latéral se situe dans une zone à une hauteur intermédiaire de l'une ou l'autre des colonnes (D1) et (D2). Le choix de la colonne (D1) ou (D2) pour le soutirage latéral est fonction de l'impureté hydrocarbonée que l'on veut extraire. Par exemple, lorsque l'impureté hydrocarbonée à extraire est le butanol, le soutirage latéral se fait dans une zone intermédiaire de la colonne (D2).

Avantageusement, le flux soutiré comprend au moins l'un des composés d'intérêt de la charge (éthanol, acétaldéhyde), à une teneur massique au moins égale à 10%, de préférence au moins égale à 20%. Ainsi, lorsque le flux soutiré ne comprend que l'un des deux composés, éthanol ou acétaldéhyde, la teneur massique en ce composé d'intérêt du flux soutiré est au moins égale à 10%, de préférence au moins égale à 20%. Lorsque le flux soutiré comprend les deux composés d'intérêt, éthanol et acétaldéhyde, la somme des teneurs massiques en éthanol et en acétaldéhyde est au moins égale à 10%, de préférence au moins égale à 20%. Le flux soutiré peut comprendre en outre de l'eau.

Avantageusement, le flux soutiré comprend l'impureté hydrocarbonée partiellement polaire considérée. La fraction massique en ladite impureté hydrocarbonée dans le flux soutiré est avantageusement comprise entre 0,5% et 50%, de préférence entre 1% et 30%.

Le flux soutiré peut être monophasique ou diphasique.

Avantageusement, la section de séparation de l'acétaldéhyde et/ou la section de séparation de l'éthanol peut(vent) également comprendre un système de reflux en tête de colonne de distillation (D1) et/ou (D2) assurant un reflux du distillat et un système de rebouillage en pied de colonne (D1) et/ou (D2). Les systèmes de reflux et de rebouillage qui peuvent être utilisés en tête en pied des colonnes (D1) et (D2) sont ceux bien connus de l'homme du métier.

Les exemples qui suivent sont présentés à titre illustratif et non limitatif du procédé de traitement selon l'invention.

### Figure(s)

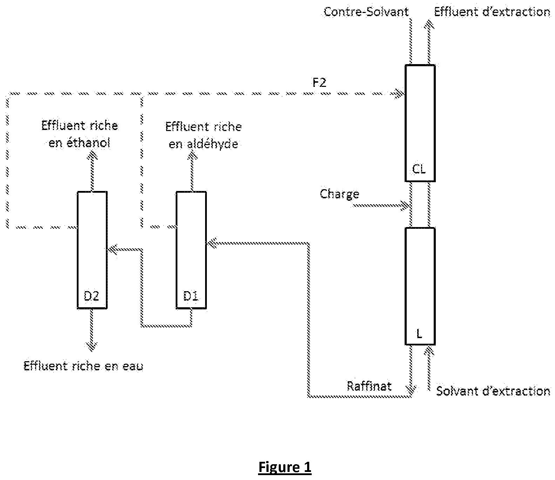

Figure 1 : Schéma du procédé selon l'invention, la section de distillation de l'étape b) comprenant un soutirage latéral sur la colonne (D1) et/ou un soutirage latéral sur la colonne (D2).
Figure 2 : Schéma du procédé selon l'invention dans le cas où l'impureté hydrocarbonée à extraire est le butanol, comme dans l'Exemple 1.

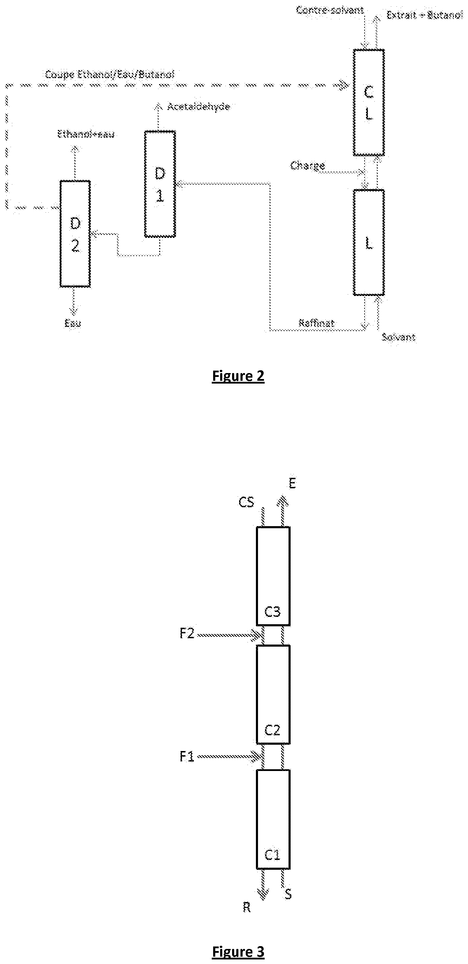

Figure 3 : Schéma du découpage de la section d'extraction dans l'Exemple 1, C1 étant la colonne de lavage (L), les zones C2 et C3 étant les zones respectivement inférieure et supérieure de la colonne de contre-lavage (CL), en-dessous et au-dessus de l'injection du flux soutiré recyclé F2, F1 étant le flux de la charge liquide à traiter, S le flux de solvant organique d'extraction, CS le flux de solvant aqueux de contre-lavage et l'effluent d'extraction (E) sortant en tête de la colonne de contre-lavage dans lequel est extrait l'impureté hydrocarbonée considérée, et le raffinat (R) sortant en fond de colonne de lavage et qui sera dirigé ensuite vers la section de distillation.
Figure 4 : Efficacité d'extraction du butanol (▲) et perte en éthanol (■) en fonction de la position d'injection F2, la position 0% représentant une injection en tête de colonne de contre-lavage (CL), et pour les deux dimensionnements de la colonne de contre-lavage (CL), 2 (traits pleins) ou 4 (traits pointillés) étages théoriques, dans le procédé de l'Exemple 1.

### EXEMPLES

### Exemple 1 - Extraction du butanol selon le procédé de l'invention

Le calcul des performances de l'extraction du butanol est réalisé en utilisant les solutions analytiques de Kremser et en divisant la colonne de contre-lavage (CL) en 2 zones (C2 et C3). Les calculs ont été réalisés en faisant varier la position de l'injection F2, c'est-à-dire le ratio des hauteurs C3/(C2+C3), C3 étant la hauteur de la zone supérieure de la colonne de contre-lavage (CL) au-dessus de l'injection F2 et C2 la hauteur de la zone inférieure de la colonne de contre-lavage (CL) au-dessous de l'injection F2. La Figure 3 schématise le découpage de la section d'extraction : C1 étant la colonne de lavage (CL) et les zones C2 et C3 étant les zones respectivement inférieure et supérieure de la colonne de contre-lavage (CL). Les calculs sont effectués en se basant sur l'hypothèse de coefficients de partage constants dans chaque zone.

Les conditions opératoires utilisées :
- nombre d'étages théoriques de la colonne de lavage (L) : 5
- nombre d'étages théoriques de la colonne de contre-lavage (CL) : 2 ou 4
- solvant organique d'extraction (solvant de lavage) (S) : hexadécane
- solvant aqueux de contre-lavage (CS) : eau
- Débits volumique Qv et massique Qm et composition massique des flux F1 (la charge liquide à traiter), F2 (le soutirage recyclé), S (le solvant organique d'extraction) et CS (le solvant aqueux de contre-lavage) : (cf. Tableau 1)

**Tableau 1 : Débits volumique Qv et massique Qm et composition massique des flux F1, F2, S et CS**

| | F1 | F2 | S | CS |
|---|---|---|---|---|
| Qv (l/h) | 1 | 0,031579 | 0.8 | 0.2 |
| Qm (kg/h) | 0,875 | 0,027632 | 0,6288 | 0,198 |
| Débit massique relatif à F1 (% poids) | 1 | 3.2% | 72% | 23% |

| Composition (% poids) : | | | | |
|---|---|---|---|---|
| Ethanol | 65,9% | 43% | 0 | 0 |
| Acétaldéhyde | 5% | 0 | 0 | 0 |
| Diéthyléther | 1% | 0 | 0 | 0 |
| Butanol | 0.5% | 9% | 0 | 0 |
| Eau | 27% | 48% | 0 | 97% |
| Hexadécane | 0% | 0 | 100% | 0 |
| Acide Acétique | 0,6% | 0 | 0 | 3% |

- Coefficients de partage (M) des composés de la charge F1 dans la zone C2 de la colonne de contre-lavage (CL) : (cf. Tableau 2).

**Tableau 2 : Coefficients de partage dans la zone C2 de la colonne de contre-lavage :**

| | M |
|---|---|
| Ethanol | 0,014 |
| Acétaldéhyde | 0,03 |
| Diéthyléther | 3,4 |
| Butanol | 0,3 |
| Eau | - |

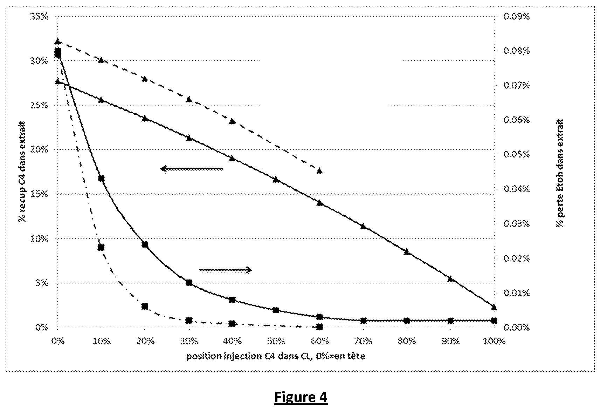

La quantité de butanol extrait, c'est-à-dire récupéré dans l'effluent d'extraction (E) en tête de colonne de contre-lavage, par rapport à la quantité massique de butanol entrant dans la section d'extraction par le flux F1, est calculée pour différentes positions de l'injection F2 sur la colonne de contre-lavage (CL), c'est-à-dire pour différents rapports C3/(C2+C3), et pour les deux dimensionnements de la colonne de contre-lavage (2 ou 4 étages théoriques), la position 0% signifiant que l'injection F2 se situe en tête de la colonne de contre-lavage (CL) et la position 100% que l'injection F2 se situe en fond de colonne de contre-lavage (CL). Simultanément, les pertes en éthanol, c'est-à-dire la quantité massique d'éthanol extrait dans l'effluent d'extraction (E) par rapport à la quantité massique d'éthanol entrant dans la section d'extraction par le flux F1, sont également calculées pour les différentes positions de l'injection F2, l'éthanol extrait dans l'effluent d'extraction (E) ne pouvant pas être recyclé dans le procédé de conversion d'éthanol en butadiène étant considéré comme perdu. Les résultats sont montrés sur la Figure 4.

D'après la Figure 4, il apparait qu'une position d'injection F2 (rapport C3/(C2+C3)) sur la colonne de contre-lavage comprise entre 10 et 30% permet d'obtenir une extraction du butanol avec une efficacité de l'ordre de 20 à 30% poids, pour une perte en éthanol négligeable (inférieure à 0,03% poids).

### Exemple 2 - Extraction de différentes impuretés hydrocarbonées

Les mêmes calculs que dans l'Exemple 1 sont réalisés pour différentes impuretés hydrocarbonées à extraire : le méthanol, le butanol, l'acétate d'éthyle. Les calculs sont effectués avec les mêmes hypothèses de départ, les mêmes débits massiques des flux que ceux de l'Exemple 1 et des compositions équivalentes (9% poids de l'impureté hydrocarbonée considérée dans la charge à traiter).

Les autres conditions opératoires utilisées sont :
- nombre d'étages théoriques de la colonne de lavage (L) : 5
- nombre d'étages théoriques de la colonne de contre-lavage (CL) : 2
- solvant organique d'extraction (solvant de lavage) (S) : hexadécane
- solvant aqueux de contre-lavage (CS) : eau
- position de l'injection F2 (C3/(C2+C3)) sur la colonne (CS) : 20%

Le Tableau 3 regroupe les températures d'ébullition des impuretés hydrocarbonées considérées (méthanol, butanol, acétate d'éthyle), ou de leur azéotrope formé avec l'eau, et leurs coefficients de partage (M) dans la zone C2 de la colonne de contre-lavage (CL).

Les efficacités d'extraction obtenues pour chacune des impuretés méthanol, butanol, acétate d'éthyle sont présentées dans le Tableau 3.

**Tableau 3 : Températures d'ébullition (Teb) (ou des azéotropes formés avec l'eau) et coefficients de partage (M) des impuretés hydrocarbonées considérées et efficacités d'extraction calculées**

| Espèce | Teb (°C) | Coefficient de partage (M) | Efficacité d'extraction (% poids extrait) |
|---|---|---|---|
| Méthanol | 65 | 0,005 | 0,02% |
| Butanol | 930°C* | 0,3 | 24% |
| Acétate d'éthyle | 70** | 2,4 | 96% |

| | | | |
|---|---|---|---|
| * : Azéotrope n-butanol/eau ** : Azéotrope acétate d'éthyle / eau | | | |

L'efficacité d'extraction est très élevée (96%) dans le cas de l'acétate d'éthyle dont le coefficient de partage dans la zone C2 de la colonne de contre-lavage (CL) est de 2,4.

Pour le butanol, l'efficacité d'extraction égale à 24% dans les conditions opératoires utilisées, est très satisfaisante. Le butanol est donc extrait partiellement, comme attendu.

A l'inverse, le méthanol dont le coefficient de partage est très faible (égal à 0,005, très inférieure à 0,1) n'est pas ou très peu extrait (0,02% poids extrait).

### Exemple 3 - Procédé de traitement de la charge sans le système de soutirage-recyclage (non-conforme)

La même charge que celle de l'Exemple 1 est traitée selon un procédé comprenant une section d'extraction liquide-liquide, avec des colonnes de lavage (L) et contre-lavage (CL) et l'hexadécane comme solvant d'extraction, et une section de distillation comprenant deux colonnes de distillation (D1) et (D2). Les colonnes de lavage (L), contre-lavage (CL) et de distillation (D1) et (D2) sont les mêmes que celles de l'Exemple 1 sont utilisées. Le procédé utilisé dans cet exemple 3 ne comprend pas soutirage dans la section de distillation ; il n'y a donc pas d'injection (F2) dans la colonne de contre-lavage.

Les autres conditions opératoires (débit et composition de la charge F1, débits et compositions des solvant et contre-solvant, coefficients de partage) sont également les mêmes que celles de l'Exemple 1.

Dans ces conditions, le taux d'extraction du butanol est de 1,8% (au lieu de 20 à 30% selon l'exemple 1).

Le butanol non extrait qui s'accumule alors dans la colonne (D2) est extrait principalement avec l'effluent éthanol en tête de colonne (D2).

## Revendications

1. Procédé de traitement, par extraction liquide-liquide et distillation, d'une charge liquide comprenant au moins de l'éthanol, de l'eau et de l'acétaldéhyde et au moins une impureté hydrocarbonée ayant une température d'ébullition comprise entre 20°C et 100°C à pression atmosphérique, ou générant, avec au moins l'un des composés de la charge liquide et/ou avec le solvant organique d'extraction et/ou avec le solvant aqueux de contre-lavage, un azéotrope dont la température d'ébullition est comprise entre 20 et 100°C à pression atmosphérique, et un coefficient de partage compris entre 0,1 et 5 en tout point de la colonne de contre-lavage (CL) de la section d'extraction, comprenant :
a) une étape d'extraction liquide-liquide comprenant une section d'extraction comprenant une colonne de lavage (L) et une colonne de contre-lavage (CL), ladite section d'extraction étant alimentée en ladite charge liquide en un point intermédiaire situé entre la tête de la colonne de lavage (L) et le fond de la colonne de contre-lavage (CL),
ladite colonne de lavage (L) étant alimentée en tête par le flux issu du fond de la colonne de contre-lavage (CL) et en fond par un flux d'un solvant organique d'extraction,
ladite colonne de contre-lavage (CL) étant alimentée en tête par un flux d'un solvant aqueux de contre lavage et en fond par le flux issu de la tête de la colonne de lavage (L),
ladite section d'extraction comprenant au moins une injection (F2) de la coupe soutirée à l'étape b), ladite injection (F2) étant située dans la moitié supérieure de la colonne de contre-lavage (CL),
ladite section d'extraction produisant en fond de colonne de lavage (L) un raffinat comprenant au moins de l'eau, de l'éthanol, de l'acétaldéhyde et une partie de ladite impureté hydrocarbonée et en tête de colonne de contre-lavage (CL) un effluent d'extraction comprenant au moins une partie de ladite impureté hydrocarbonée ;
b) une étape de distillation comprenant :
∘ au moins une étape de séparation de l'acétaldéhyde comprenant une section de séparation de l'acétaldéhyde, composée au moins d'une colonne de distillation (D1) alimentée par le raffinat issu de l'étape a) dans une zone intermédiaire de la colonne (D1) et produisant en tête un effluent riche en acétaldéhyde et en fond un effluent eau/éthanol ; et
∘ au moins une étape de séparation de l'éthanol comprenant une section de séparation de l'éthanol, composée au moins d'une colonne de distillation (D2) alimentée par l'effluent eau/éthanol issu du fond de la colonne (D1) dans une zone intermédiaire de la colonne (D2), et produisant en tête un effluent riche en éthanol et en fond un effluent riche en eau,
∘ au moins un soutirage latéral sur la colonne (D1) ou la colonne (D2) d'une coupe comprenant au moins l'un des composés éthanol ou acétaldéhyde, à une fraction massique d'au moins 10% de la masse totale de la coupe soutirée, et ladite impureté hydrocarbonée, à une fraction massique comprise entre 0,5% et 50% de la masse totale de la coupe soutirée, le soutirage latéral étant situé dans une zone intermédiaire de la colonne (D1) ou (D2).

2. Procédé de traitement selon la revendication 1, dans lequel la charge comprend moins de 5% poids d'impureté hydrocarbonée.

3. Procédé de traitement selon la revendication 1 ou 2, dans lequel l'impureté hydrocarbonée a un coefficient de partage compris entre 0,2 et 2 en tout point de la colonne de contre-lavage (CL) de la section d'extraction.

4. Procédé de traitement selon l'une quelconque des revendications précédentes, dans lequel l'injection (F2) est située dans la partie haute de la colonne de contre-lavage (CL) de telle sorte que la distance entre la tête de la colonne de contre-lavage (CL) et l'injection (F2) est égale à une longueur comprise entre 10 et 30% de la longueur totale de la colonne de contre-lavage (CL).

5. Procédé de traitement selon l'une quelconque des revendications précédentes, dans lequel le flux recyclé (F2) est injecté à l'étape a) à un débit massique compris entre 0,1% et 10% du débit massique de la charge liquide alimentant la section d'extraction.

6. Procédé de traitement selon l'une quelconque des revendications précédentes, dans lequel le flux recyclé (F2) comprend l'impureté hydrocarbonée à une concentration massique comprise entre 0,5% et 50%.

7. Procédé de traitement selon l'une quelconque des revendications précédentes, dans lequel le débit massique en solvant organique d'extraction à l'étape a) est compris entre 25% et 200% du débit massique de la charge liquide alimentant la section d'extraction.

8. Procédé de traitement selon l'une quelconque des revendications précédentes, dans lequel le débit massique de solvant aqueux à l'étape a) est compris entre 10% et 80% du débit massique de la charge liquide alimentant la section d'extraction.

9. Procédé de traitement selon l'une quelconque des revendications précédentes, dans lequel l'étape d'extraction liquide-liquide est opérée à une température comprise entre 10°C et 70°C, à une pression comprise entre 01 MPa et 0,5 MPa et avec des temps de séjour compris entre 0,5 et 10h dans la colonne de lavage et compris entre 0,5 et 6h dans la colonne de contre-lavage.

## Patentansprüche

1. Verfahren zur Behandlung, mittels flüssig/flüssig-Extraktion und Destillation, einer flüssigen Charge, die zumindest Ethanol, Wasser und Acetaldehyd sowie mindestens eine kohlenwasserstoffartige Verunreinigung umfasst, welche eine Siedetemperatur im Bereich von 20 °C bis 100 °C unter Atmosphärendruck hat oder, mit mindestens einer der Verbindungen der flüssigen Charge und/oder mit dem organischen Extraktionslösungsmittel und/oder mit dem wässrigen Lösungsmittel zum Gegenwaschen, ein Azeotrop bildet, dessen Siedetemperatur im Bereich von 20 bis 100 °C bei Atmosphärendruck liegt, und welche einen Verteilungskoeffizienten hat, der an jedweder Stelle der Gegenwaschkolonne (CL) des Extraktionsabschnitts im Bereich von 0,1 bis 5 liegt, wobei es Folgendes umfasst:
a) einen Schritt der flüssig/flüssig-Extraktion, welcher einen Extraktionsabschnitt umfasst, der eine Waschkolonne (L) und eine Gegenwaschkolonne (CL) umfasst, wobei dem Extraktionsabschnitt die flüssige Charge an einer Zwischenstelle zugeführt wird, welche sich zwischen dem Kopf der Waschkolonne (L) und dem Sumpf der Gegenwaschkolonne (CL) befindet,
wobei der Waschkolonne (L) kopfseitig der Stoffstrom, welcher aus dem Sumpf der Gegenwaschkolonne (CL) stammt, sowie sumpfseitig ein Stoffstrom eines organischen Extraktionslösungsmittels zugeführt wird,
wobei der Gegenwaschkolonne (CL) kopfseitig ein Stoffstrom eines wässrigen Gegenwaschlösungsmittels sowie sumpfseitig der Stoffstrom zugeführt wird, welcher aus dem Kopf der Waschkolonne (L) stammt,
wobei der Extraktionsabschnitt mindestens eine Stelle (F2) zum Einleiten der Fraktion umfasst, welche im Schritt b) entnommen wurde, wobei sich die Einleitstelle (F2) in der oberen Hälfte der Gegenwaschkolonne (CL) befindet,
wobei in dem Extraktionsabschnitt sumpfseitig der Waschkolonne (L) ein Raffinat, welches zumindest Wasser, Ethanol, Acetaldehyd und eine Teilmenge der kohlenwasserstoffartigen Verunreinigung umfasst, und kopfseitig der Gegenwaschkolonne (CL) ein Extraktionsstoffstrom erzeugt wird, welcher mindestens eine Teilmenge der kohlenwasserstoffartigen Verunreinigung umfasst;
b) einen Destillationsschritt, der Folgendes umfasst:
o mindestens einen Schritt des Abtrennens des Acetaldehyds, wobei er einen Abschnitt zur Abtrennung des Acetaldehyds umfasst, der sich aus mindestens einer Destillationskolonne (D1) zusammensetzt, welcher das Raffinat, welches aus dem Schritt a) stammt, in einem Zwischenbereich der Kolonne (D1) zugeführt wird, wobei sie weiterhin kopfseitig einen Stoffstrom, welcher reich an Acetaldehyd ist, und sumpfseitig einen Wasser/Ethanol-Stoffstrom erzeugt; und
o mindestens einen Schritt des Abtrennens des Ethanols, wobei er einen Abschnitt zur Abtrennung des Ethanols umfasst, der sich aus mindestens einer Destillationskolonne (D2) zusammensetzt, welcher der Wasser/Ethanol-Stoffstrom, welcher aus dem Sumpf der Kolonne (D1) stammt, in einem Zwischenbereich der Kolonne (D2) zugeführt wird, wobei sie weiterhin kopfseitig einen Stoffstrom, welcher reich an Ethanol ist, und sumpfseitig einen Stoffstrom erzeugt, welcher reich an Wasser ist,
o mindestens eine seitliche Stelle an der Kolonne (D1) oder der Kolonne (D2) zur Entnahme einer Fraktion, die mindestens eine der Verbindungen Ethanol oder Acetaldehyd, wobei deren Massenanteil an der Gesamtmasse der entnommenen Fraktion mindestens 10 % beträgt, sowie die kohlenwasserstoffartige Verunreinigung umfasst, wobei deren Massenanteil an der Gesamtmasse der entnommenen Fraktion im Bereich von 0,5 % bis 50 % liegt, wobei sich die seitliche Entnahmestelle in einem Zwischenbereich der Kolonne (D1) oder (D2) befindet.

2. Behandlungsverfahren nach Anspruch 1, wobei die Charge weniger als 5 Gewichts-% an kohlenwasserstoffartiger Verunreinigung umfasst.

3. Behandlungsverfahren nach Anspruch 1 oder 2, wobei die kohlenwasserstoffartige Verunreinigung an jedweder Stelle der Gegenwaschkolonne (CL) des Extraktionsabschnitts einen Verteilungskoeffizienten im Bereich von 0,2 bis 2 hat.

4. Behandlungsverfahren nach einem beliebigen der vorhergehenden Ansprüche, wobei sich die Einleitstelle (F2) derart im oberen Teil der Gegenwaschkolonne (CL) befindet, dass der Abstand zwischen dem Kopf der Gegenwaschkolonne (CL) und der Einleitstelle (F2) von einer Länge ist, welche 10 bis 30 % der Gesamtlänge der Gegenwaschkolonne (CL) ausmacht.

5. Behandlungsverfahren nach einem beliebigen der vorhergehenden Ansprüche, wobei der zurückgeführte Stoffstrom (F2) im Schritt a) mit einem Massendurchsatz eingeleitet wird, welcher 0,1 % bis 10 % des Massendurchsatzes der flüssigen Charge ausmacht, welche dem Extraktionsabschnitt zugeführt wird.

6. Behandlungsverfahren nach einem beliebigen der vorhergehenden Ansprüche, wobei der zurückgeführte Stoffstrom (F2) die kohlenwasserstoffartige Verunreinigung in einer Massenkonzentration im Bereich von 0,5 % bis 50 % umfasst.

7. Behandlungsverfahren nach einem beliebigen der vorhergehenden Ansprüche, wobei im Schritt a) der Massendurchsatz an organischem Extraktionslösungsmittel 25 % bis 200 % des Massendurchsatzes der flüssigen Charge ausmacht, welche dem Extraktionsabschnitt zugeführt wird.

8. Behandlungsverfahren nach einem beliebigen der vorhergehenden Ansprüche, wobei im Schritt a) der Massendurchsatz an wässrigem Lösungsmittel 10 % bis 80 % des Massendurchsatzes der flüssigen Charge ausmacht, welche dem Extraktionsabschnitt zugeführt wird.

9. Behandlungsverfahren nach einem beliebigen der vorhergehenden Ansprüche, wobei der Schritt der flüssig/flüssig-Extraktion bei einer Temperatur im Bereich von 10 °C bis 70 °C, bei einem Druck im Bereich von 0,1 MPa bis 0,5 MPa und mit einer Verweildauer im Bereich von 0,5 bis 10 Std. in der Waschkolonne und im Bereich von 0,5 bis 6 Std. in der Gegenwaschkolonne betrieben wird.

## Claims

1. Process for the treatment by liquid-liquid extraction and distillation of a liquid feedstock comprising at least ethanol, water and acetaldehyde and at least one hydrocarbon-based impurity with a boiling point of between 20°C and 100°C at atmospheric pressure, or generating, with at least one of the compounds of the liquid feedstock and/or with the organic extraction solvent and/or with the aqueous back-washing solvent, an azeotrope whose boiling point is between 20 and 100°C at atmospheric pressure, and a partition coefficient of between 0.1 and 5 at any point in the back-washing (BW) column of the extraction section, comprising:
a) a liquid-liquid extraction step comprising an extraction section comprising a washing (W) column and a back-washing (BW) column, said extraction section being fed with said liquid feedstock at an intermediate point located between the top of the washing (W) column and the bottom of the back-washing (BW) column,
said washing (W) column being fed at the top with the stream originating from the bottom of the back-washing (BW) column and at the bottom with a stream of an organic extraction solvent,
said back-washing (BW) column being fed at the top with a stream of an aqueous back-washing solvent and at the bottom with the stream originating from the top of the washing (W) column,
said extraction section comprising at least one injection (F2) of the cut withdrawn in step b), said injection (F2) being located in the top half of the back-washing (BW) column,
said extraction section producing, at the bottom of the washing (W) column, a raffinate comprising at least water, ethanol, acetaldehyde and a portion of said hydrocarbon-based impurity and, at the top of the back-washing (BW) column, an extraction effluent comprising at least a portion of said hydrocarbon-based impurity;
b) a distillation step comprising:
o at least one acetaldehyde separation step comprising an acetaldehyde separation section, composed of at least one distillation column (D1) fed with the raffinate originating from step a) in an intermediate zone of the column (D1) and producing at the top an acetaldehyde-rich effluent and at the bottom a water/ethanol effluent; and
o at least one ethanol separation step comprising an ethanol separation section, composed of at least one distillation column (D2) fed with the water/ethanol effluent originating from the bottom of the column (D1) in an intermediate zone of the column (D2), and producing at the top an ethanol-rich effluent and at the bottom a water-rich effluent,
o at least one side withdrawal on the column (D1) or the column (D2) of a cut comprising at least one of the compounds ethanol or acetaldehyde, at a mass fraction of at least 10% of the total mass of the withdrawn cut, and said hydrocarbon-based impurity, at a mass fraction of between 0.5% and 50% of the total mass of the withdrawn cut, the side withdrawal being located in an intermediate zone of the column (D1) or (D2).

2. Treatment process according to Claim 1, in which the feedstock comprises less than 5% by weight of hydrocarbon-based impurity.

3. Treatment process according to Claim 1 or 2, in which the hydrocarbon-based impurity has a partition coefficient of between 0.2 and 2 at any point in the back-washing (BW) column of the extraction section.

4. Treatment process according to any one of the preceding claims, in which the injection (F2) is located in the top part of the back-washing (BW) column such that the distance between the top of the back-washing (BW) column and the injection (F2) is equal to a length of between 10% and 30% of the total length of the back-washing (BW) column.

5. Treatment process according to any one of the preceding claims, in which the recycled stream (F2) is injected into step a) at a mass flow rate of between 0.1% and 10% of the mass flow rate of the liquid feedstock feeding the extraction section.

6. Treatment process according to any one of the preceding claims, in which the recycled stream (F2) comprises the hydrocarbon-based impurity at a mass concentration of between 0.5% and 50%.

7. Treatment process according to any one of the preceding claims, in which the mass flow rate of organic extraction solvent in step a) is between 25% and 200% of the mass flow rate of the liquid feedstock feeding the extraction section.

8. Treatment process according to any one of the preceding claims, in which the mass flow rate of aqueous solvent in step a) is between 10% and 80% of the mass flow rate of the liquid feedstock feeding the extraction section.

9. Treatment process according to any one of the preceding claims, in which the liquid-liquid extraction step is performed at a temperature of between 10°C and 70°C, at a pressure of between 0.1 MPa and 0.5 MPa and with residence times of between 0.5 and 10 hours in the washing column and between 0.5 and 6 hours in the back-washing column.
